(19) Europäisches Patentamt
European Patent Office
Office européen des brevets

(11) EP 0 665 217 B1

(12) **FASCICULE DE BREVET EUROPEEN**

(45) Date de publication et mention de la délivrance du brevet:
**07.01.1998 Bulletin 1998/02**

(51) Int. Cl.$^{6}$: **C07C 309/31**, C10M 105/72, C10M 135/10, C07G 17/00, C10M 159/24, B01F 17/12

(21) Numéro de dépôt: **95400113.7**

(22) Date de dépôt: **20.01.1995**

# (54) Nouveaux composés sulfonés du bismuth, leur préparation et leur utilisation notamment pour la préparation de produits colloidaux surbasés, eux-mêmes utilisables comme additifs pour lubrifiants

Sulfonierte Wismutverbindungen, ihre Herstellung und ihre Verwendung zur Herstellung von überbasischen kolloidalen Produkten, verwendbar als Zusätze für Schmiermittel

Sulphonated bismuth compounds, their preparation and their use notably for the preparation of overbased colloidal products, themselves suitable for use as additives for lubricants

(84) Etats contractants désignés:
**BE DE ES GB IT NL**

(30) Priorité: **26.01.1994 FR 9400928**
**03.03.1994 FR 9402574**

(43) Date de publication de la demande:
**02.08.1995 Bulletin 1995/31**

(73) Titulaire:
**INSTITUT FRANCAIS DU PETROLE**
**92502 Rueil-Malmaison (FR)**

(72) Inventeurs:
- **Delfort, Bruno**
  **F-75005 Paris (FR)**
- **Born, Maurice**
  **F-92000 Nanterre (FR)**
- **Daoudal, Bertrand**
  **F-89230 Pontigny (FR)**
- **Lallement, Jacques**
  **F-93300 Aubervilliers (FR)**

(56) Documents cités:
**FR-A- 2 154 806** **FR-A- 2 451 364**

- **INDIAN JOURNAL OF CHEMISTRY, SECTION A, vol. 29A, page 982 P. KAPOOR, ET AL.: 'Preparation and characterisation of para-toluenesulphonato complexes of arsenic(III), antimony(III) and bismuth(III)'**

EP 0 665 217 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Xerox (UK) Business Services
2.15.8/3.4

## Description

L'invention concerne de nouveaux composés de type sulfonate de bismuth, leur préparation et leurs utilisations.

Dans diverses applications, il peut être intéressant de disposer de produits colloïdaux de type "sulfonates surbasés" renfermant du bismuth.

Or, jusqu'à présent, ce type de produits colloïdaux sont formés principalement d'un coeur de carbonate de calcium ou de magnésium (voire de borate de calcium), maintenu en suspension colloïdale par un tensioactif consistant généralement en un alkylarylsufonate du même métal, calcium ou magnésium, ou dans certains cas de sodium ou de potassium.

On a maintenant découvert de nouveaux composés du bismuth de type alkylarylsulfonate ayant des propriétés tensioactives et pouvant être utilisés notamment comme produits de départ dans la fabrication de produits colloïdaux.

La préparation de certains sulfonates de bismuth a déjà été décrite notamment dans INDIAN JOURNAL OF CHEMISTRY, Vol. 29 A 982-985 (1990). Dans cet article, sont décrits des para-toluène sulfonates de bismuth préparés dans des solvants polaires tels que le nitrométhane l'acétonitrile ou le diéthyléther. Les auteurs décrivent la préparation d'un sulfonate de bismuth de type $(RSO_3)_3Bi$ par l'un des processus réactionnels suivants:

(1)

$$3RSO_3Ag + BiCl_3 \rightarrow (RSO_3)_3Bi + 3AgCl$$

et
(2)

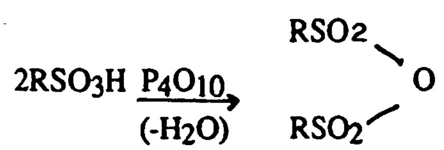


suivi de

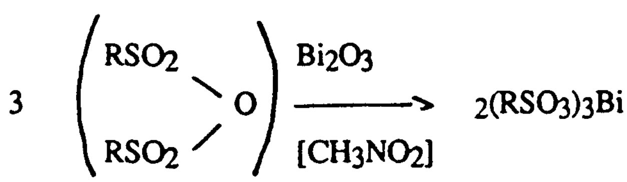


Ils décrivent également la préparation d'un composé de bismuth de type $RSO_3BiO$ par la réaction :

$$2RSO_3H + Bi_2O_3 \rightarrow 2RSO_3BiO + H_2O$$

(dans les formules ci-dessus R représente un radical para -tolyle

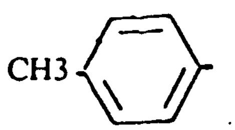
).

Les produits considérés sont insolubles dans les hydrocarbures et ne possèdent aucune propriété tensioactive (ils ne présentent pas de HLB = équilibre hydrophile-lipophile).

On a maintenant découvert qu'il était possible d'obtenir sélectivement, à partir d'un même composé sulfonique de départ, des produits de structures analogues à celles des sulfonates de bismuth de l'art antérieur mais solubles dans les hydrocarbures, présentant des propriétés tensioactives et pouvant être utilisés notamment comme additifs dans les lubrifiants et dans les carburants. Ils peuvent encore servir de produits intermédiaires dans la préparation de produits colloïdaux utilisables comme additifs dans les lubrifiants. Les produits de l'invention sont préparés par des méthodes qui ne sont pas analogues à celles décrites dans l'art antérieur. Les produits eux-mêmes et leur méthodes de préparation font partie de l'invention.

Les composés sulfonés du bismuth selon l'invention sont définis d'une manière générale par le fait qu'ils répondent à l'une des formules suivantes :

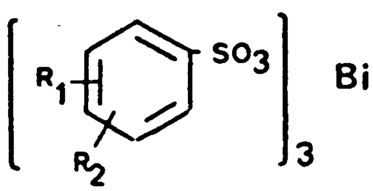

I

et

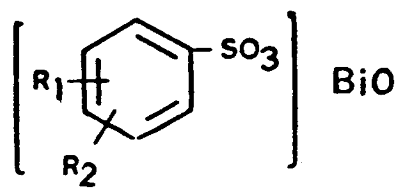

II

où $R^1$ représente un radical hydrocarboné aliphatique monovalent linéaire ou ramifié renfermant de 8 à 36 atomes de carbone et $R^2$ représente un atome d'hydrogène ou un radical hydrocarboné aliphatique monovalent, linéaire ou ramifié, renfermant de 8 à 36 atomes de carbone.

Une même formule englobant les formules générales (I) et (II) des produits de l'invention pourrait être la suivante :

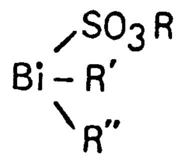

avec

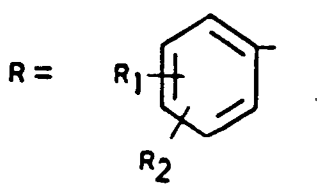

R' et R" peuvent être chacun $RSO_3$- , auquel cas on retrouve la formule (I) ci-dessus; ou alors

R' et R" peuvent représenter ensemble un atome d'oxygène, auquel cas on retrouve la formule (II) ci-dessus.

Les modes opératoires permettant de préparer sélectivement les produits de formules générales (I) et (II) à partir de même acide sulfonique $RSO_3H$ se différencient l'un de l'autre principalement par le fait que l'on opère en éliminant l'eau de réaction formée au cours de l'opération pour obtenir un produit de formule générale (I), et en présence d'eau pour obtenir un produit de formule générale (II).

Le procédé de préparation des composés sulfonés de bismuth de l'invention comprend la réaction d'au moins un acide sulfonique de formule générale :

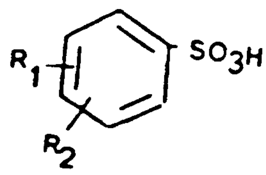

avec de l'oxyde de bismuth $Bi_2O_3$.

L'acide sulfonique est en général mis en jeu en solution dans un solvant hydrocarboné aromatique aliphatique ou cycloaliphatique éventuellement substitué par exemple par des halogènes (tel que par exemple le toluène, un xylène, un chlorobenzène ou le cyclohexane).

La proportion d'oxyde de bismuth mise en jeu est en général de 1/6 à 2/1 en moles par rapport à l'acide sulfonique. La réaction est conduite en l'absence ou en présence d'eau.

Si l'on opère en l'absence d'eau, pour préparer un composé sulfoné de bismuth de formule générale (I), on ajoute éventuellement au mélange réactionnel un monoalcool aliphatique léger renfermant par exemple de 1 à 6 atomes de carbone : le plus souvent le méthanol ; puis on chauffe de manière à chasser le monoalcool aliphatique éventuellement présent, et l'on poursuit le chauffage par exemple à la température de reflux du solvant hydrocarboné, en général à une température supérieure à 100°C, ce pendant un temps par exemple de 0,5 à 5 heures.

L'eau est éliminée au cours de la réaction généralement par distillation hétéroazéotropique ou éventuellement en présence d'un anhydride tel que l'anhydride acétique. On laisse ensuite en général refroidir le mélange réactionnel, on en sépare les éventuels produits solides, par exemple par filtration, et on évapore le solvant sous pression réduite.

Dans ce cas, on obtient le composé sulfoné de bismuth répondant à la formule (I)

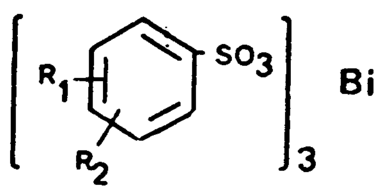

I

Pour préparer un composé sulfoné du bismuth de formule générale (II)

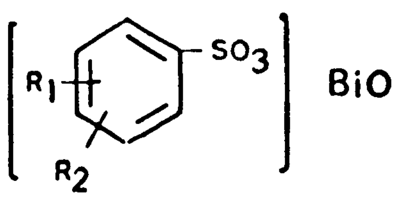

II

on opère en présence d'eau. Comme décrit précédemment, on fait réagir au moins un acide sulfonique de formule générale

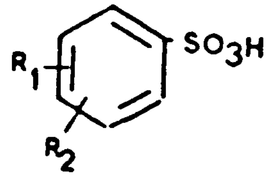

avec de l'oxyde de bismuth, en général en une proportion molaire de 1/6 à 2/1 par rapport à l'acide sulfonique, au sein d'un solvant hydrocarboné aromatique, aliphatique ou cycloaliphatique, éventuellement halogéné. On ajoute au mélange réactionnel une quantité d'eau pouvant aller jusqu'à environ 50 % en masse par rapport à l'acide sulfonique, et éventuellement un promoteur consistant par exemple en un monoalcool aliphatique de 1 à 6 atomes de carbone. De préférence le mélange réactionnel est maintenu dans un premier temps à une température inférieure à 100°C, par exemple pendant un temps de 15 à 60 minutes, puis porté à une température supérieure à 100°C, par exemple pendant un temps de 0,5 à 3 heures.

Les composés de l'invention peuvent être caractérisés notamment par leur teneur en bismuth qui peut aller jusqu'à environ 25 % en masse pour les produits de formule générale (I) et jusqu'à environ 45 % en masse pour les produits de formule générale (II), suivant la nature de l'acide sulfonique utilisé.

Les composés de l'invention ont des propriétés de tensioactifs et peuvent être utilisés comme tels dans diverses applications. Ils trouvent en particulier une application comme additifs pour huiles lubrifiantes et comme additifs carburants sans plomb, où ils peuvent avoir une action anti-récession sur les sièges de soupapes des moteurs à combustion interne à essence. Ils peuvent encore servir de produits de départ dans la fabricattion de produits colloïdaux surbasés utilisables notamment comme additifs pour les lubrifiants.

Dans cette utilisation, on prépare lesdits produits colloïdaux surbasés par un procédé qui comprend la carbonatation au moyen d'anhydride carboniqe, d'au moins un oxyde ou hydroxyde de calcium, de baryum ou de magnésium, le carbonate de calcium, de baryum ou de magnésium étant maintenu à l'état de dispersion colloïdal au sein d'un milieu organique par un composé sulfoné du bismuth répondant à l'une des formules générales :

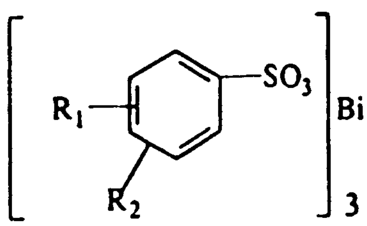
et
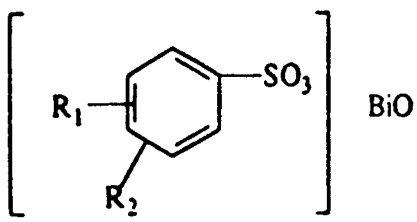

I

II

dans lesquelles $R^1$ représente un radical hydrocarboné aliphatique monovalent, linéaire ou ramifié, renfermant de 8 à 36 atomes de carbone et $R^2$ représente un atome d'hydrogène ou un radical hydrocarboné aliphatique monovalent, linéaire ou ramifié, renfermant de 8 à 36 atomes de carbone. De préférence, $R^1$ représente un radical tétracosyle et $R^2$ représente un atome d'hydrogène.

La réaction de carbonatation effectuée selon toute technique usuelle de préparation de produits surbasiques, comme celles décrites par exemple dans les brevets US 2.865.956, 3.150.088, 3.537.996, 3.830.739, 3.865.737, 4.148.740, 3.953.519, 3.966.621, et 4.505.830 et le brevet français 2.101.813, en remplaçant les sulfonates alcalins ou alcalino-terreux généralement utilisés par les composés sulfonés du bismuth décrits plus haut.

On peut signaler qu'il existe des variantes de la réaction de surbasification qui font notamment appel à des carbonates préformés à partir d'alcoxydes et de $CO_2$ avant la mise en contact avec le sel alcalin ou alcalino-terreux du composé acide; elles sont décrites notam-ment dans les brevets US 2.956.018, 3.932.289 et 4.104.180.

Les produits colloïdaux surbasés obtenus dans cette application selon l'invention sont stables, solubles dans les lubrifiants minéraux et synthétiques.

Ils présentent une réserve de basicité représentée par un indice total de base (TBN) pouvant aller jusqu'à environ 550 mg KOH/g (c'est-à-dire environ 10 meq basique/g de produit.

Leur teneur en bismuth peut aller jusqu'à 40 % en masse et leur teneur en calcium, en baryum ou en magnésium, peut aller par exemple jusqu'à environ 20 % en masse.

Le caractère colloïdal de ces produits est vérifié par dialyse à travers une membrane en latex. Les analyses de bismuth localisent cet élément dans la fraction n'ayant pas dialysé (concentrat), qui constitue la partie colloïdale du produit.

Les composés colloïdaux surbasés renfermant du bismuth obtenus constituent d'excellents additifs antiusure et extrême-pression. Les additifs antiusure et extrême-pression sont incorporés aux lubrifiants lorsque ceux-ci sont destinés à lubrifier des organes soumis à des contraintes mécaniques importantes, tels que la distribution dans les moteurs thermiques, les engrenages, les roulements ou les butées. Des contraintes mécaniques importantes apparaissent également lors de l'usinage des métaux, qu'il s'agisse de coupe ou de formage.

En outre, ces composés colloïdaux surbasés renfermant du bismuth sont doués d'une grande stabilité thermique ce qui autorise leur emploi dans les lubrifiants soumis en service à des températures très élevées pouvant atteindre 160 °C, comme dans certains carters de moteurs sévères, dans des transmissions très chargées ou des coupes de métaux à grande vitesse.

Dans l'utilisation de ces produits comme additifs pour huiles lubrifiantes et graisses, on peut les incorporer à celles-ci par exemple à une concentration de 0,1 à 25 % en masse, de préférence de 1 à 15 % en masse.

Les huiles lubrifiantes (ou les graisses) contiennent en outre en général un ou plusieurs autres additifs tels que les additifs améliorant l'indice de viscosité, des additifs d'abaissement du point d'écoulement, des antioxydants, des antirouille, des additifs anticorrosion du cuivre, des détergents, des antiusure, des antimousse, des dispersants, des réducteurs de frottements, avec lesquels les produits de l'invention sont compatibles.

Les exemples suivants illustrent la préparation de composés sulfonés du bismuth selon l'invention.

Exemple 1 :

Dans un réacteur équipé d'un agitateur et d'un séparateur de Dean et Stark, on introduit 32,6 g (0,1 mole) d'acide dodécylbenzènesulfonique que l'on dissout dans 150 $cm^3$ de xylène. On introduit alors 23,3 g (0,05 mole) d'oxyde de bismuth $Bi_2O_3$ puis 16 $cm^3$ de méthanol. On porte le milieu à la température de reflux du xylène après avoir chassé le méthanol et on maintient le milieu à cette température pendant 2 heures. Après retour à la température ambiante, on procède à la filtration du milieu puis à l'évaporation du filtrat sous pression réduite. On obtient un produit homogène renfermant 17,8 % en masse de bismuth (théorie pour $(C_{12}H_{25} - C_6H_4 - SO_3)_3$ Bi = 17,65 %).

Exemple 2 :

Dans un réacteur équipé d'un agitateur et d'un séparateur de Dean et Stark, on introduit 200 g d'un acide alkylarylsulfonique de masse molaire équivalente à 700 (soit 0,286 équivalent acide) que l'on dissout dans 500 $cm^3$ de xylène. On introduit alors 68,0 g (0,146 mole) d'oxyde de bismuth $Bi_2O_3$ puis 46 $cm^3$ de méthanol. On porte le milieu à la température de reflux du xylène après avoir chassé le méthanol et on maintient le milieu à cette température pendant 2 heures. Après retour à la température ambiante, on procède à la filtration du milieu puis à l'évaporation du filtrat sous pression réduite. On obtient un produit homogène renfermant 9,4 % en masse de bismuth (théorie pour $(R\text{-}SO_3)_3$ Bi = 9,1 %).

Exemple 3 :

Dans un réacteur équipé d'un agitateur et d'un séparateur de Dean et Stark, on introduit 32,6 g (0,1 mole) d'acide dodecylbenzènesulfonique que l'on dissout dans 150 $cm^3$ de xylène. On introduit alors 46,6 g (0,10 mole) d'oxyde de bismuth $Bi_2O_3$, 10,5 g (0,58 mole) d'eau, puis 56 $cm^3$ de méthanol. On porte le milieu à la température de 70°C pendant 2 heures puis après distillation du méthanol et de l'eau présente dans le milieu, on maintient celui-ci à la température de reflux du xylène pendant 2 heures. Après retour à la température ambiante, on procède à la filtration du milieu puis à l'évaporation du filtrat sous pression réduite. On obtient un produit homogène renfermant 37,6 % en masse de bismuth (théorie pour $(C_{12}H_{25} - C_6H_4 - SO_3)BiO$ = 38,0 %).

Exemple 4 :

Dans un réacteur équipé d'un agitateur et d'un séparateur de Dean et Stark, on introduit 500 g d'un acide alkylarylsulfonique de masse molaire moyenne équivalente à 700 (soit 0,714 équivalent acide) que l'on dissout dans 800 $cm^3$ de xylène. On introduit alors 199 g (0,427 mole) d'oxyde de bismuth $Bi_2O_3$, 30,0 g (1,67 mole) d'eau puis 162 $cm^3$ de méthanol. On porte le milieu à la température de 70°C pendant 2 heures puis après distillation du méthanol et de l'eau présente dans le milieu, on maintient celui-ci à la température de reflux du xylène pendant 2 heures. Après retour à la température ambiante, on procède à la filtration du milieu puis à l'évaporation du filtrat sous pression réduite. On obtient un produit homogène renfermant 24,1 % en masse de bismuth (théorie pour $RSO_3$ BiO = 22,6 %).

Exemple 5 :

Dans un réacteur équipé d'un agitateur, d'un dispositif d'introduction d'anhydride carbonique gazeux et d'un thermomètre, on introduit 73,50 g d'un alkylarylsulfonate de bismuth obtenu comme décrit dans l'exemple 2, contenant 9,4 % en masse de bismuth que l'on dissout dans 219,9 g d'une huile minérale 130 Neutral, 800 $cm^3$ de xylène, 30 $cm^3$ de méthanol, 1,2 $cm^3$ d'eau et 2,7 $cm^3$ d'ammoniaque. On disperse dans cette solution 67,80 g (0,916 mole) de chaux $Ca(OH)_2$ et sous une vigoureuse agitation on introduit par barbotage 32,3 g (0,733 mole) d'anhydride carbonique

gazeux en 40 minutes à une température n'excédant pas 45 °C. Après décantation et élimination de la phase supérieure hydroalcoolique on procède à la filtration de la phase inférieure suivie de l'évaporation des solvants sous pression réduite. On obtient un produit liquide homogène dont les caractéristiques sont les suivantes:

réserve alcaline = 3,46 meq basique/g; indice total de base (TBN) = 194 mg KOH/g,
Bi = 2,45 % en masse,
Ca = 7,50 % en masse,
matière active = 39,2 % en masse
huile = 60,8 % en masse.

Exemple 6

Dans un réacteur équipé d'un agitateur, d'un dispositif d'introduction d'anhydride carbonique gazeux et d'un thermomètre, on introduit 74,20 g d'un alkylarylsulfonate de bismuth contenant 24,1 % en masse de bismuth obtenu comme décrit dans l'exemple 4 que l'on dissout dans 166,9 g d'une huile minérale 130 Neutral, 800 $cm^3$ de xylène, 30 $cm^3$ de méthanol, 1,3 $cm^3$ d'eau et 2,6 $cm^3$ d'ammoniaque. On disperse dans cette solution 34,3 g (0,464 mole) de chaux $Ca(OH)_2$ et sous une vigoureuse agitation on introduit par barbotage 16,3 g (0,371 mole) d'anhydride carbonique gazeux en 40 minutes à une température n'excédant pas 45 °C. Après décantation et élimination de la phase supérieure hydroalcoolique on procède à la filtration de la phase inférieure suivie de l'évapora-tion des solvants sous pression réduite. On obtient un produit liquide homogène dont les caractéristiques sont les suivantes :

réserve alcaline = 2,17 meq basique/g; indice total de base (TBN) = 122 mg KOH/g,
Bi = 7,95 % en masse,
Ca = 4,50 % en masse,
matière active = 40,5 % en masse,
huile = 59,5 % en masse.

Exemple 7- Examen des produits par dialyse dans l'heptane à travers une membrane en latex

Les produits des exemples 5 et 6 précédents sont soumis à une dialyse en solution dans l'heptane normal à travers une membrane en latex. Pour chaque essai, on détermine la fraction massique ayant dialysé (dialysat) et celle n'ayant pas dialysé (concentrat), cette dernière constituant la partie colloïdale. On détermine également pour chaque fraction la concentration en bismuth. Les résultats sont rassemblés dans le tableau n° 1. La localisation du bismuth exclusivement dans les concentrats confirme le caractère colloïdal des produits considérés.

Tableau 1

| Examen des produits par dialyse | | | | |
|---|---|---|---|---|
| Produit de l'exemple | Concentrat | | Dialysat | |
| | % en masse | Bi % | % en masse | Bi % |
| 5 | 39,2 | 6,1 | 60,8 | 0 |
| 6 | 40,5 | 19,9 | 59,5 | 0 |

Exemple 8 - Évaluation des propriétés antiusure et extrême-pression

Les produits des exemples 5 et 6 de l'invention sont évalués pour leurs propriétés anti-usure et extrême-pression dans une huile lubrifiante 130 Neutral. Les performances sont évaluées sur machine 4 billes selon la méthode ASTM D 2783. Les résultats sont rassemblés dans le tableau n° 2.

Tableau 2

| Évaluation des propriétés anti-usure et extrême-pression des produits à 7 % de matière active dans l'huile 130 Neutral | |
|---|---|
| Produit de l'exemple | Charge de soudure (daN) |
| néant | 126 |
| 5 | 220 |
| 6 | 220 |

## Revendications

**1.** Composé sulfoné de bismuth caractérisé en ce qu'il répond à l'une des formules générales :

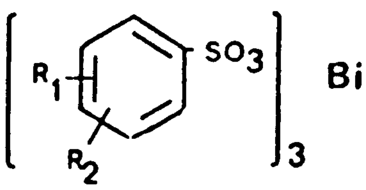

I

et

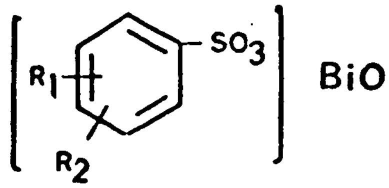

II

dans lesquelles $R^1$ représente un radical hydrocarboné aliphatique monovalent, linéaire ou ramifié, renfermant de 8 à 36 atomes de carbone et $R^2$ représente un atome d'hydrogène ou un radical hydrocarboné aliphatique monovalent, linéaire ou ramifié renfermant de 8 à 36 atomes de carbone.

**2.** Composé selon la revendication 1, caractérisé en ce que, dans chacune des formules (I) et (II), $R^1$ représente un radical dodécyle et $R^2$ représente un atome d'hydrogène.

**3.** Procédé de préparation d'un composé sulfoné de bismuth selon la revendication 1 ou 2 et répondant à la formule (I), caractérisé en ce que l'on met à réagir au moins un acide sulfonique de formule générale

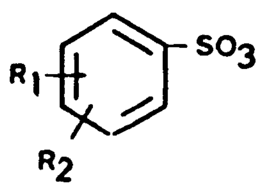

où $R^1$ et $R^2$ sont définis comme dans la revendication 1 ou 2, en solution dans un solvant hydrocarboné éventuellement halogéné, avec de l'oxyde de bismuth $Bi_2O_3$ ; on chauffe le mélange réactionnel et on sépare le composé sulfoné de bismuth répondant à la formule (I).

**4.** Procédé selon la revendication 3 caractérisé en ce que la proportion molaire de l'oxyde de bismuth est de 1/6 à 2/1 par rapport à l'acide sulfonique.

**5.** Procédé selon l'une des revendications 3 et 4, caractérisé en ce que le mélange réactionnel est chauffé à une température supérieure à 100°C.

6. Procédé selon l'une des revendications 3 à 5 caractérisé en ce que l'on introduit dans le mélange réactionnel avant chauffage,à titre de promoteur, un monoalcool aliphatique de 1 à 6 atomes de carbone.

7. Procédé de préparation d'un composé sulfoné de bismuth selon la revendication 1 ou 2 et répondant à la formule (II), caractérisé en ce que l'on met à réagir au moins un acide sulfonique de formule générale

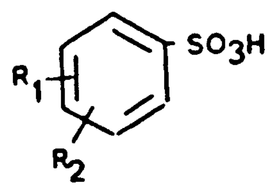

où $R^1$ et $R^2$ sont définis comme dans la revendication 1 ou 2, en solution dans un solvant hydrocarboné éventuellement halogéné, avec de l'oxyde de bismuth $Bi_2O_3$, on ajoute de l'eau, on chauffe le mélange réactionnel et on sépare le composé sulfoné de bismuth répondant à la formule (II).

8. Procédé selon la revendication 7, caractérisé en ce que la proportion molaire d'oxyde de bismuth est de 1/6 à 2/1 par rapport à l'acide sulfonique.

9. Procédé selon l'une des revendication 7 et 8, caractérisé en ce que le mélange réactionnel est d'abord chauffé à une température inférieure à 100°C puis à une température supérieure à 100°C.

10. Procédé selon l'une des revendications 7 à 9 caractérisé en ce que l'on introduit dans le mélange réactionnel avant chauffage, à titre de promoteur, un monoalcool aliphatique de 1 à 6 atomes de carbone.

11. Utilisation d'un composé sulfoné du bismuth selon l'une des revendications 1 et 2 ou obtenu par un procédé selon l'une des revendications 3 à 10 comme agent tensioactif.

12. Utilisation d'un composé sulfoné de bismuth selon l'une des revendication 1 et 2 ou obtenu par un procédé selon l'une des revendications 3 à 10 comme additif pour carburants de type essence et comme additif pour huiles lubrifiantes.

13. Utilisation d'un composé sulfoné de bismuth selon l'une des revendications 1 et 2 ou obtenu par un procédé selon l'une des revendications 3 à 10, dans la préparation de produits colloïdaux surbasés par un procédé comprenant la carbonatation d'au moins un oxyde ou hydroxyde de calcium, de baryum ou de magnésium, le carbonate de calcium, de baryum ou de magnésium formé étant maintenu à l'état de dispersion colloïdal au sein d'un milieu organique par un composé sulfoné du bismuth répondant à l'une des formules générales :

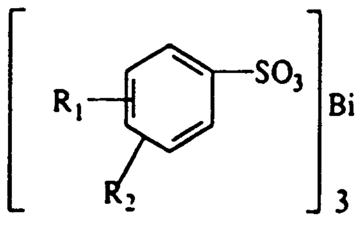

I

et

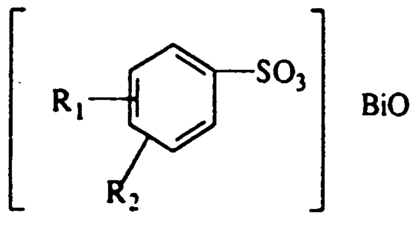

II

dans lesquelles $R^1$ représente un radical hydrocarboné aliphatique monovalent, linéaire ou ramifié, renfermant de 8 à 36 atomes de carbone et $R^2$ représente un atome d'hydrogène ou un radical hydrocarboné aliphatique monovalent, linéaire ou ramifié, renfermant de 8 à 36 atomes de carbone.

14. Produit colloïdal surbasé obtenu dans l'utilisation selon la revendication 13.

15. Produit colloïdal surbasé selon la revendication 14, caractérisé en ce que, dans chacune des formules (I) et (II) du

composé sulfoné de bismuth, $R^1$ représente un radical tétraccosyle et $R^2$ représente un atome d'hydrogène.

**16.** Produit colloïdal surbasé selon l'une des revendications 14 et 15, caractérisé en ce qu'il présente une réserve alcaline représentée par un indice total de base allant jusqu'à envrion 550 mg KOH/g de produit (environ 10 meq basique/g).

**17.** Produit colloïdal surbasé selon l'une des revendications 14 à 16, caractérisé par une teneur du bismuth allant jusqu'à environ 40% en masse.

**18.** Produit colloïdal surbasé selon l'une des revendications 14 à 17, caractérisé par une teneur en calcium, en baryum ou en magnésium allant jusqu'à environ 20% en masse.

**19.** Utilisation d'un produit colloïdal surbasé selon l'une des revendications 14 à 18, comme additif pour huiles ou graisses lubrifiantes.

**20.** Utilisation selon la revendication 19 caractérisée en ce que ledit produit colloïdal surbasé est ajouté à l'huile ou à la graisse lubrifiante à une concentration de 0,1 à 25% en masse.

## Claims

**1.** Sulfonated bismuth compound, characterized in that said compound corresponds to one of the general formulae

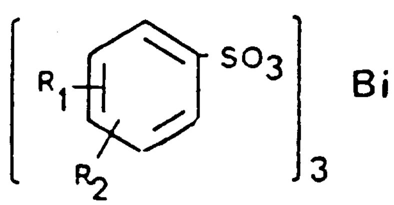

I

et

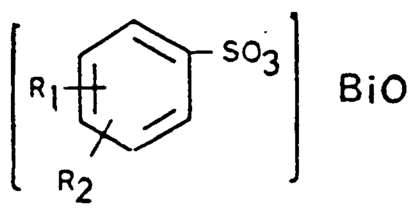

II

in which $R_1$ represents a monovalent linear or branched aliphatic hydrocarbon radical containing 8 to 36 atoms of carbon, and $R_2$ represents an atom of hydrogen or a monovalent linear or branched aliphatic hydrocarbon radical containing 8 to 36 atoms of carbon.

**2.** Compound according to claim 1, characterized that, in each formulae of (I) and (II), $R_1$ represents a dodecyl radical, and $R_2$ represents an atom of hydrogen.

**3.** Process for preparation of a sulfonated bismuth compound according to claim 1 or 2 and corresponding to formula (I) characterized in that at least one sulfonic acid corresponding to the general formula

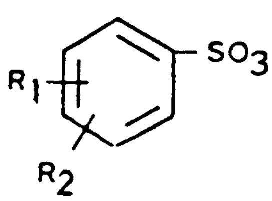

in which $R_1$ and $R_2$ are as defined in claim 1 or 2, is reacted, in solution in a hydrocarbon solvent, optionally halogenated, with bismuth oxide, $Bi_2O_3$ ; the reactive mixture is heated and the sulfonated bismuth compound corresponding to formula (I) is separated.

**4.** Process according to claim 3, characterized in that the molar proportion of the bismuth oxide in relation to the sulfonic acid is 1/6 to 2/1.

5. Process according one of claims 3 and 4, characterized in that the reactive mixture is heated to a temperature of more than 100°C.

6. Process according to one of claim 3 to 5, characterized in that an aliphatic monohydric alcohol containing 1 to 6 atoms of carbon is added as a catalyst promoter to the reactive mixture before heating.

7. Process for preparation of a sulfonated bismuth compound according to claim 1 or 2 and corresponding to formula (II) characterized in that at least one sulfonic acid corresponding to the general formula

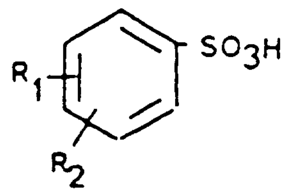

where $R_1$ and $R_2$ are as defined in claim 1 or 2, is reacted, in solution in a hydrocarbon solvent, optionally halogenated, with bismuth oxide, $Bi_2O_3$ ; water is added to the reactive mixture; the reactive mixture is heated, and the sulfonated bismuth compound corresponding to formulae (II) is separated.

8. Process according to claim 7, characterized in that the molar proportion of the bismuth oxide in ralation the the sulfonic acid is 1/6 to 2/1.

9. Process according to one of claims 7 and 8 characterized in that the reactive mixture is first heated to a temperature of less than 100°C, then to a temperature greater than 100°C.

10. Process according to any of claims 7 to 9, characterized an aliphatic monohydric alcohol having 1 to 6 atoms of carbon is added as a catalyst promoter to the reactive mixture before heating.

11. The use of a sulfonated bismuth compound according to one of claims 1 or 2 or obtained by a process according to one of claims 3 to 10 as a surface active agent.

12. The use of a sulfonated bismuth compound according to one of claims 1 and 2 or obtained by a process according to one of claims 3 to 10, as an additive for fuel of the gasoline type and as an additive for lubricating oils.

13. The use of a sulfonated bismuth compound according to one of claims 1 and 2, or obtained by a process according to one of claims 3 to 10, in the preparation of overbased colloidal product by a process comprising carbonating at least one oxide or hydroxide of calcium, barium, or magnesium, the carbonate of said calcium, barium or magnesium being maintained in form of a colloidal dispersion within an organic medium by a sulfonated bismuth compound corresponding to one to the general formulae

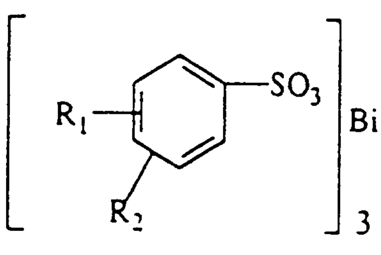

et

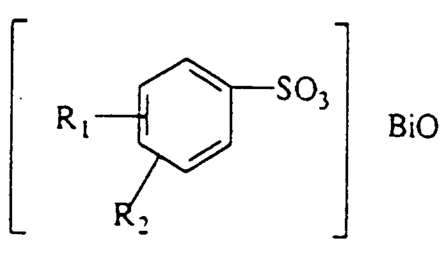

I

II

in which $R_1$ represents a monovalent linear or branched aliphatic hydrocarbon radical containing 8 to 36 atoms of carbon, and $R_2$ represents an atom of hydrogen or a monovalent linear or branched aliphatic hydrocarbon radical

containing 8 to 36 atoms of carbon.

**14.** Overbased colloidal product obtained by the process according to claim 13.

**15.** Overbased colloidal product according to claim 14, characterized in that in each of formulae (I) and (II) of the sulfonated bismuth compound, $R_1$ represents a tetracosyl radical and $R_2$ represents an atom of hydrogen.

**16.** Overbased colloidal product according to one of claims 14 and 15, characterized in that it has an alkaline reserve as represented by a total base number of up to approximately 550 mg KOH/g of product (approximately 10 basic meq/g).

**17.** Overbased colloidal product according to one of claims 14 to 16 characterized by a bismuth content of up to approximately 40 % by weight.

**18.** Overbased colloidal product according to one of claims 14 to 17 characterized by a calcium, barium, or magnesium content of up to approximately 20 % by weight.

**19.** The use of an overbased colloidal product according to one of claims 14 to 18 as an additive for lubricating oils or greases.

**20.** Use according to claim 19, characterized in that said overbased colloidal product is added to the lubricating oil or grease at a concentration of from 0.1 to 25 % by weight.

**Patentansprüche**

**1.** Sulfonierte Bismuthverbindung, dadurch gekennzeichnet, daß sie einer der allgemeinen Formeln (I) und (II):

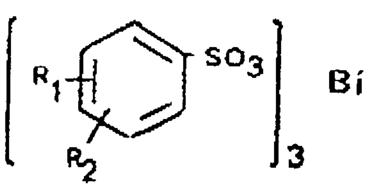

I

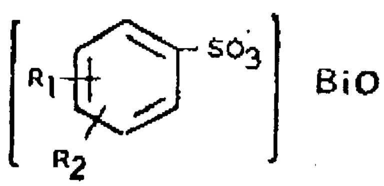

II

entspricht, in denen $R^1$ ein monovalentes, lineares oder verzweigtes aliphatisches Kohlenwasserstoffradikal mit 8 bis 36 Kohlenstoffatomen und $R^2$ ein Wasserstoffatom oder ein monovalentes, lineares oder verzweigtes aliphatisches Kohlenwasserstoffradikal mit 8 bis 36 Kohlenstoffatomen darstellt.

**2.** Verbindung nach Anspruch 1, dadurch gekennzeichnet, daß in jeder der Formeln (I) und (II) $R^1$ ein Dodekylradikal und $R^2$ ein Wasserstoffatom darstellt.

**3.** Verfahren zur Herstellung einer sulfonierten Bismuthverbindung nach Anspruch 1 oder 2 und gemaß der Formel (I), dadurch gekennzeichnet, daß man wenigstens eine Sulfonsäure der allgemeinen Formel

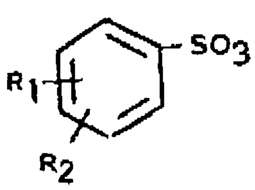

,

wo $R^1$ und $R^2$ wie in Anspruch 1 oder 2 definiert sind, in Lösung in einem eventuell halogenierten Kohlenwasserstofflösungsmittel mit Bismuthoxid $Bi_2O_3$ zur Reaktion bringt; das Reaktionsgemisch erwärmt und die der Formel (I) entsprechende sulfonierte Bismuthverbindung abtrennt.

4. Verfahren nach Anspruch 3, dadurch gekennzeichnet, daß das molare Verhältnis des Bismuthoxids 1/6 bis 2/1 in bezug auf die Sulfonsäure ist.

5. Verfahren nach einem der Ansprüche 3 oder 4, dadurch gekennzeichnet, daß das Reaktionsgemisch auf eine Temperatur über 100°C erwärmt wird.

6. Verfahren nach einem der Ansprache 3 bis 5, dadurch gekennzeichnet, daß in das Reaktionsgemisch vor der Erwärmung ein aliphatischer Monoalkohol mit 1 bis 6 Kohlenstoffatomen als Promotor eingegeben wird.

7. Verfahren zur Herstellung einer sulfonierten Bismuthverbindung nach Anspruch 1 oder 2 und gemäß der Formel (II), dadurch gekennzeichnet, daß man wenigstens eine Sulfonsäure der allgemeinen Formel

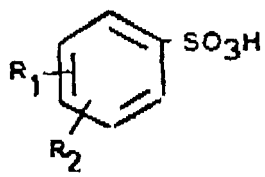

,

wo $R^1$ und $R^2$ wie in Anspruch 1 oder 2 definiert sind, in Lösung in einem eventuell halogenierten Kohlenwasserstofflösungsmittel mit Bismuthoxid $Bi_2O_3$ zur Reaktion bringt, Wasser hinzufügt, das Reaktionsgemisch erwärmt und die der Formel (II) entsprechende sulfonierte Bismuthverbindung abtrennt.

8. Verfahren nach Anspruch 7, dadurch gekennzeichnet, daß das molare Verhältnis des Bismuthoxids 1/6 bis 2/1 in bezug auf die Sulfonsäure ist.

9. Verfahren nach einem der Ansprüche 7 und 8, dadurch gekennzeichnet, daß das Reaktionsgemisch zunächst auf eine Temperatur unter 100°C und dann auf eine Temperatur über 100°C erwärmt wird.

10. Verfahren nach einem der Ansprüche 7 bis 9, dadurch gekennzeichnet, daß in das Reaktionsgemisch vor der Erwärmung ein aliphatischer Monoalkohol mit 1 bis 6 Kohlenstoffatomen als Promotor eingegeben wird.

11. Verwendung einer sulfonierten Bismuthverbindung, nach einem der Ansprüche 1 und 2 oder erhalten durch ein Verfahren nach einem der Ansprüche 3 bis 10, als grenzflächenaktiver Stoff.

12. Verwendung einer sulfonierten Bismuthverbindung, nach einem der Ansprüche 1 und 2 oder erhalten durch ein Verfahren nach einem der Ansprüche 3 bis 10, als Zusatz für benzinartige Brennstoffe und als Zusatz für Schmieröle.

13. Verwendung einer sulfonierten Bismuthverbindung, nach einem der Ansprüche 1 und 2 oder erhalten durch ein Verfahren nach einem der Ansprüche 3 bis 10, in der Herstellung von superbasischen kolloidalen Erzeugnissen durch ein Verfahren, das die Sattigung mit Kohlensäure wenigstens eines Oxids oder Hydroxids von Kalzium, Barium oder Magnesium umfaßt, wobei das gebildete Kalzium-, Barium- oder Magnesiumkarbonat im Zustand einer kolloidalen Dispersion in einem organischen Medium durch eine sulfonierte Bismuthverbindung nach einer der allgemeinen Formeln (I) und (II)

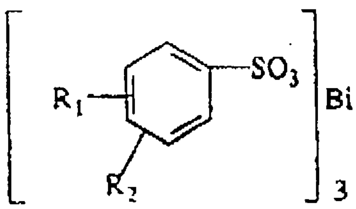 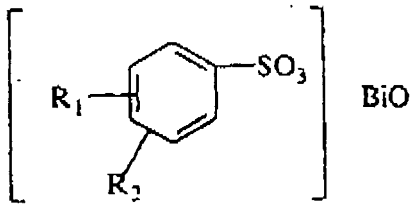

I II

gehalten wird, in denen $R^1$ ein monovalentes, lineares oder verzweigtes aliphatisches Kohlenwasserstoffradikal mit 8 bis 36 Kohlenstoffatomen und $R^2$ ein Wasserstoffatom oder ein monovalentes, lineares oder verzweigtes aliphatisches Kohlenwasserstoffradikal mit 8 bis 36 Kohlenstoffatomen darstellt.

**14.** Superbasisches kolloidales Erzeugnis, erhalten in der Anwendung nach Anspruch 13.

**15.** Superbasisches kolloidales Erzeugnis nach Anspruch 14, dadurch gekennzeichnet, daß in jeder der Formeln (I) und (II) der sulfonierten Bismuthverbindung $R^1$ ein Tetraeicosylradikal und $R^2$ ein Wasserstoffatom darstellt.

**16.** Superbasisches kolloidales Erzeugnis nach einem der Ansprüche 14 und 15, dadurch gekennzeichnet, daß es eine Alkalinitätsreserve, dargestellt durch einen Gesamtbasenindex von bis zu 550 mgKOH/g Erzeugnis (ca. 10 Millibasenäquivalent/g) aufweist.

**17.** Superbasisches kolloidales Erzeugnis nach einem der Ansprüche 14 bis 16, gekennzeichnet durch einen Bismuthgehalt von bis zu 40 Gew.-%.

**18.** Superbasizches kolloidalez Erzeugnis nach einem der Ansprüche 14 bis 17, gekennzeichnet durch einen Kalzium-, Barium- oder Magnesiumgehalt von bis zu 20 Gew.-%.

**19.** Verwendung eines superbasischen kolloidalen Erzeugnisses nach einem der Ansprüche 14 bis 18 als Zusatz für Schmieröle oder -fette.

**20.** Verwendung nach Anspruch 19, dadurch gekennzeichnet, daß das superbasische kolloidale Erzeugnis dem Schmieröl oder -fett mit einer Konzentration von 0,1 bis 25 Gew.-% zugesetzt wird.